(19) Europäisches Patentamt
European Patent Office
Office européen des brevets

(11) EP 3 244 882 B1

(12) **FASCICULE DE BREVET EUROPEEN**

(45) Date de publication et mention de la délivrance du brevet:
**29.04.2020 Bulletin 2020/18**

(21) Numéro de dépôt: **15704040.3**

(22) Date de dépôt: **12.01.2015**

(51) Int Cl.:
*A61K 31/122* (2006.01) *A61K 31/192* (2006.01)
*A61K 31/7034* (2006.01) *A61K 36/68* (2006.01)
*C07C 59/84* (2006.01) *A61P 29/00* (2006.01)
*A61P 17/00* (2006.01) *A61P 19/02* (2006.01)
*A61P 11/14* (2006.01) *A61K 8/35* (2006.01)
*A61K 8/36* (2006.01) *A61K 8/365* (2006.01)
*A61K 8/60* (2006.01) *A61K 8/97* (2017.01)
*A61Q 7/00* (2006.01) *A61Q 15/00* (2006.01)
*A61K 9/00* (2006.01) *A61Q 19/00* (2006.01)
*A61Q 19/06* (2006.01) *A61Q 19/08* (2006.01)
*A61Q 19/10* (2006.01)

(86) Numéro de dépôt international:
**PCT/FR2015/050064**

(87) Numéro de publication internationale:
**WO 2016/113473 (21.07.2016 Gazette 2016/29)**

(54) **COMPOSÉS O-QUINONIQUES COMME AGENTS NEUTRALISANT L'OXYDE NITRIQUE**

O-CHINON-VERBINDUNGEN ALS MITTEL ZUR NEUTRALISATION VON STICKOXID

O-QUINONE COMPOUNDS AS AGENTS NEUTRALISING NITRIC OXIDE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(43) Date de publication de la demande:
**22.11.2017 Bulletin 2017/47**

(73) Titulaire: **Institut Des Substances Végétales**
**63000 Clermont-Ferrand (FR)**

(72) Inventeurs:
- **JEAN, Daniel**
  **F-63270 Vic-Le-Comte (FR)**
- **POULIGON, Maryse**
  **F-63800 Cournon-d'Auvergne (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**WO-A1-02/07720 FR-A1- 2 733 419**
**FR-A1- 2 733 419**

- **Boskabady ET AL: "Antitussive Effect of Plantago lanceolata in Guinea Pigs", IJMS Iran J Med Sci, 1 septembre 2006 (2006-09-01), page 143, XP55586789, Extrait de l'Internet: URL:http://ijms1.sums.ac.ir/article_40040_d878c6269305f6d3dff099ed39937418.pdf [extrait le 2019-05-08]**
- **European Medicines Agency: "Assessment report on Plantago lanceolata L., follium", , 22 novembre 2011 (2011-11-22), pages 1-24, XP002743888, Extrait de l'Internet: URL:http://www.ema.europa.eu/docs/en_GB/document_library/Herbal_-_HMPC_assessment_report/2012/02/WC500123351.pdf [extrait le 2015-09-03]**
- **Boskabady ET AL: "Antitussive Effect of Plantago lanceolata in Guinea Pigs", IJMS Iran J Med Sci, 1 September 2006 (2006-09-01), page 143, XP55586789, Retrieved from the Internet: URL:http://ijms1.sums.ac.ir/article_40040_d878c6269305f6d3dff099ed39937418.pdf [retrieved on 2019-05-08]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 244 882 B1

## Description

La présente invention se rapporte au domaine du traitement de maladies et/ou désordres inflammatoires résultant d'un excès d'oxyde nitrique (NO) ; elle a plus particulièrement pour objet des composés o-quinoniques comme agents neutralisants l'oxyde nitrique et leur utilisation thérapeutique ou cosmétique.

L'oxyde nitrique (NO) est une des molécules les plus petites produites par les systèmes biologiques. Ses actions sont aussi diverses dans les organismes que les cellules capables de la produire. En effet, son statut de radical libre relativement instable que lui confère sa structure électronique (sa demi-vie est de 5 secondes) lui permet d'agir sur un grand nombre de molécules plus ou moins sensibles à son action au sein des processus métaboliques cellulaires et extracellulaires.

Il a été montré dans les années 80 que l'oxyde nitrique agissait de façon spécifique en tant que médiateur de la dynamique vasculaire en pilotant la relaxation des vaisseaux. Entre autres cellules, au cours des processus inflammatoires, cette molécule est produite par les cellules endothéliales (1,2) et les macrophages (3,4) par des enzymes, les nitric oxide synthases (NOs), catalysant la transformation de l'arginine en citrulline. Ces enzymes existent de façon constitutionnelle ou sont induites lors de réactions immunitaires, on parle alors de iNOs. La vasodilatation est induite via la stimulation de la guanylate cyclase du muscle lisse (5). L'oxyde nitrique est aussi formé par les cellules endothéliales en réponse à une variété de substances comme la bradykinine (2), l'histamine et la 5-hydroxytryptamine (6). L'oxyde nitrique peut également être formé par les macrophages activés par des lipopolysaccharides ou des cytokines (6).

L'oxyde nitrique joue également un rôle dans l'immunité grâce à sa forte réactivité chimique qui lui permet de participer à la lyse des germes phagocytés par les macrophages. En présence du radical super-oxyde, l'oxyde nitrique se transforme en peroxynitrite, lui même agent particulièrement agressif comme oxydant.

L'induction de la synthèse de l'oxyde nitrique a été montrée au cours de la réponse inflammatoire initiée par des produits microbiens lors d'infections ou lors de réactions auto-immunes. L'oxyde nitrique est donc un médiateur de la physiologie qui possède une face positive en régulant la vasodilatation et en participant à la fonction immunitaire, et une face négative lorsqu'il est émis en trop grande quantité.

Présent en excès, il peut produire une inflammation, une destruction des cellules et des tissus et peut également provoquer une vasodilatation induisant de la douleur ou une hypotension fatale lors des chocs septiques ou anaphylactiques.

Il a notamment été montré que l'oxyde nitrique avait une influence sur la prolifération des kératinocytes (7) et de très forts niveaux d'oxyde nitrique plasmatique ont été observés chez des sujets présentant un psoriasis (8), suggérant l'influence de ce médiateur sur cette maladie.

L'atopie est une prédisposition à la réaction amplifiée de la réponse immunitaire. Sous sa forme dermatologique, la dermatite atopique affecte environ 15% des enfants des pays développés. Il a été montré qu'une production élevée d'oxyde nitrique est impliquée dans l'inflammation, la vasodilatation et les dommages oxydatifs aux cellules et aux tissus des peaux des sujets présentant une dermatite atopique (9).

L'oxyde nitrique est également impliqué dans l'érythème solaire (10).

De même, au niveau articulaire, l'oxyde nitrique est impliqué dans les manifestations inflammatoires de l'arthrite. Il a été également démontré dans des modèles animaux que l'administration d'inhibiteurs de NOs réduit significativement l'inflammation du cartilage (22).

Concernant la réponse inflammatoire systémique à laquelle se réfère le choc septique, elle produit une vasorelaxation généralisée due à la production massive de l'oxyde nitrique, qui se traduit par une hypotension pouvant conduire au décès en l'absence de traitement approprié appliqué en urgence (11). Ce mécanisme est également celui qui se développe lors du choc anaphylactique (12).

En conséquence, s'il peut être parfois souhaitable d'apporter de l'oxyde nitrique à un organe qui en manque comme c'est le cas par exemple dans l'hypertension chronique associée à des suites d'infarctus du myocarde ou pour le prévenir, dans de nombreux autres cas, il serait nécessaire de neutraliser ce même oxyde nitrique afin de réduire ses effets nocifs, soit de façon ambulatoire comme dans la dermatite atopique, l'eczéma, le psoriasis, la toux, ou l'arthrose, soit de façon massive en urgence pour traiter les collapsus associés au choc septique ou anaphylactique.

La production d'oxyde nitrique peut être réduite en inhibant les nitric oxide synthases (NOs), enzymes qui le libèrent à partir de la L-arginine, ou bien en neutralisant l'oxyde nitrique de façon chimique ou en le captant avec des complexants appropriés.

On connaît des inhibiteurs de NOs, parmi eux des dérivés de l'arginine, comme les dérivés méthylés, nitrés ou propylés de cet acide aminé (13), mais leur usage en thérapeutique systémique comporte de graves inconvénients (14). En effet, l'inhibition des NOs est difficilement contrôlable et pour éviter un surdosage pouvant conduire à de l'hypertension pulmonaire, il est souvent nécessaire d'administrer de l'oxyde nitrique gazeux par voie respiratoire en même temps que l'inhibiteur de NOs, tout en surveillant les paramètres cardiovasculaires du patient. Cet inconvénient pourrait être dû au manque de spécificité des inhibiteurs employés vis-à-vis des différentes isoformes des NOs, mais pour l'instant, aucun inhibiteur spécifique n'a fait la preuve de son efficacité et de sa fiabilité dans des études cliniques.

Des inhibiteurs de NOs ont été proposés pour traiter des troubles esthétiques de la peau (15, 16), des maladies impliquant une activité inflammatoire ou des réponses à des cytokines pro-inflammatoires (23) mais leur utilisation est limitée par les effets secondaires néfastes, tels l'hypertension artérielle, qu'ils peuvent manifester et le fait que ces inhibiteurs soient tous des produits de synthèse alors que les consommateurs préfèrent aujourd'hui se tourner vers des substances d'origine naturelle.

Il existe aussi des molécules capables de neutraliser l'oxyde nitrique, comme l'hémoglobine qui est considérée comme la molécule de référence dans ce domaine, et diverses molécules de synthèse comme le carboxy-2-phenyl-4,4,5,5-tetramethylimidazoline-1-oxyl-3-oxide (carboxy-PTIO) (17) et des dérivés du ruthénium (18). L'utilisation de toutes ces molécules comporte des inconvénients en particulier pour des applications thérapeutiques et cosmétiques ; à titre d'exemple, les dérivés du ruthénium sont considérés comme potentiellement toxiques et carcinogènes.

Mis à part ces trois groupes de molécules, un très grand nombre d'autres composants d'origine végétale ont été proposés comme possibles agents neutralisants de l'oxyde nitrique, comme des flavonoïdes et plus généralement des polyphénols. Mais l'évaluation de leur effet neutralisant par la méthode de Griess qui mesure l'ion $NO_2$-, ne permet pas d'évaluer l'effet réel de la molécule testée. En effet, tout antioxydant capable d'abaisser la concentration en radicaux oxygénés dans le milieu est un inhibiteur de la réaction qui conduit de NO à l'ion $NO_2$-, et donc la méthode de Griess fournit une indication erronée de l'effet réel de la molécule sur son pouvoir neutralisant de l'oxyde nitrique, car il est impossible de faire la distinction entre le pouvoir antioxydant et le pouvoir neutralisant vis-à-vis de l'oxyde nitrique.

Dans le but d'avoir une appréciation réelle de l'effet neutralisant de composé vis-à-vis de l'oxyde nitrique, les Inventeurs ont développé une méthode spécifique *in vitro* qui évalue directement la concentration de l'oxyde nitrique dans le milieu et qui en mesure la neutralisation en temps réel en présence des molécules testées ; l'exemple I décrit cette méthode.

Pour cela, l'oxyde nitrique est préparé dans un récipient séparé en réalisant la réduction du nitrite de sodium par le sulfate ferreux et entrainé par un courant d'azote gazeux dans un autre récipient contenant le produit à tester en solution dans un tampon. Dans cette solution est placée une sonde ampérométrique qui mesure en temps réel le courant produit par la présence d'oxyde nitrique en solution.

Une fois munis de cette méthode spécifique d'évaluation de la neutralisation d'oxyde nitrique, les Inventeurs ont pu confirmer l'effet neutralisant de certaines molécules connues comme telles, comme l'hémoglobine, mais découvert aussi de façon surprenante que certaines molécules, décrites comme de bons neutralisants de l'oxyde nitrique ne l'étaient pas en réalité, une fois passées au crible de cette méthode spécifique, notamment des flavonoïdes comme la quercétine, la catéchine et la lutéoline.

Ils ont également identifié des molécules qui s'avèrent de très bons neutralisants de l'oxyde nitrique et qui n'avaient jamais été décrites comme telles.

Ainsi, la présente invention vise l'identification de composés neutralisants efficacement l'oxyde nitrique, facilement utilisables en thérapeutique ou en cosmétique et ne présentant pas les inconvénients de ceux décrits précédemment.

La présente divulgation décrit un composé de formule (I) :

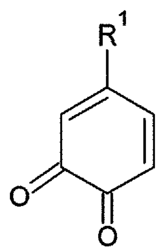

(I)

dans laquelle $R^1$ est sélectionné parmi le groupe comprenant :

- $-CH=CH-COOR^2$

  avec $R^2$ pouvant représenter :

  - H, une chaîne alkyle en $C_1$-$C_3$ linéaire, éventuellement substituée par une ou plusieurs fonctions choisies parmi les fonctions hydroxyle et acide carboxylique, par un cycle benzénique, par un diphénol ou par un radical caffeoyl ;

- Un cycle en $C_6$ saturé ou insaturé, éventuellement substitué par une ou plusieurs fonctions choisies parmi les fonctions hydroxyle et acide carboxylique et/ou par un radical caffeoyl ;
- La formule -Y-O-X-(3,4)diphénol (II) avec :
  - Y est un cycle pyranose, substitué par un $-CH_2-OH$ et au moins une fonction hydroxyle et éventuellement substitué par un rhamnose ;
  - X pouvant représenter la chaîne $-CH_2-CH_2-$ ou $-CH_2-CH(OH)-$ ;

•

$-Z-O-R^3$

Avec :

- Z pouvant représenter la chaîne $-CH_2-CH_2-$, $-CH(OH)-CH_2-$ ou $-CH_2-CH(COOH)-$;
- $R^3$ pouvant représenter :
  - -CO-CH=CH-(3,4)diphénol ou
  - La formule -W-O-CO-CH=CH-(3,4)diphénol (III) avec W étant un cycle pyranose, substitué par un $-CH_2-OH$ et au moins une fonction hydroxyle et éventuellement substitué par un rhamnose ;
  pour son utilisation pour la prévention et/ou le traitement de maladies et/ou désordres résultant d'un excès de NO.

**[0024]** Par physiologiquement acceptable, on entend compatible avec l'administration à un sujet, de préférence un mammifère, par toute voie d'administration.

L'excès de NO peut être déterminé par la mesure du taux de nitrates dans le sérum sanguin (9). On considère alors qu'un individu humain présente un excès de NO lorsque sa concentration en nitrates dans le sérum sanguin est supérieure ou égale à 14 mmol/L, en particulier 30 mmol/L ou encore 50 mmol/L.

Selon l'invention, le composé de formule (I) est tel que $R^1$ est sélectionné parmi le groupe comprenant :

•

$-CH=CH-COOR^2$

avec $R^2$ représentant la formule -Y-O-X-(3,4)diphénol (II) avec :

- Y représente un cycle pyranose, substitué par un $-CH_2-OH$ et au moins une fonction hydroxyle et substitué par un rhamnose ;
- X représente la chaîne $-CH_2-CH_2-$ ;

•

$-Z-O-R^3$

Avec :

- Z représente la chaîne $-CH_2-CH_2-$ ;
- $R^3$ représente la formule-W-O-CO-CH=CH-(3,4)diphénol (III) avec W représente un cycle pyranose, substitué par un $-CH_2-OH$ et au moins une fonction hydroxyle et substitué par un rhamnose.

Selon une variante de la présente divulgation, celle-ci se rapporte à un composé de formule (Ia) tel que :

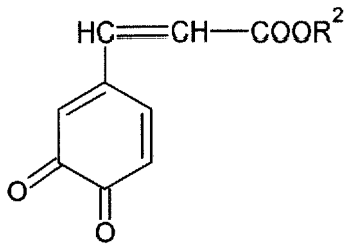

(Ia)

avec $R^2$ pouvant représenter :

- H, une chaîne alkyle en $C_1$-$C_3$ linéaire, éventuellement substituée par une ou plusieurs fonctions choisies parmi les fonctions hydroxyle et acide carboxylique, par un cycle benzénique, par un diphénol ou par un radical caffeoyl ;
- Un cycle en $C_6$ saturé ou insaturé, éventuellement substitué par une ou plusieurs fonctions choisies parmi les fonctions hydroxyle et acide carboxylique et/ou par un radical caffeoyl ;
- La formule -Y-O-X-(3,4)diphénol (II) avec :

  - Y est un cycle pyranose, substitué par un -$CH_2$-OH et au moins une fonction hydroxyle et éventuellement substitué par un rhamnose ;
  - X pouvant représenter la chaîne -$CH_2$-$CH_2$- ou -$CH_2$-CH(OH)-.

Selon une variante de l'invention, celle-ci se rapporte à un composé de formule (I) tel que :

- $R^1$ est -CH=CH-COOR$^2$ et $R^2$ répond à la formule -Y-O-X-(3,4)diphénol (II) avec Y est le glucose substitué par un rhamnose et X représente la chaîne -$CH_2$-$CH_2$- ; ou
- $R^1$ est -Z-O-R$^3$ avec Z représentant la chaine -$CH_2$-$CH_2$-, ; et $R^3$ représente la formule -W-O-CO-CH=CH-(3,4)diphénol (III) avec W est un glucose substitué par un rhamnose.

Selon une variante préférée de l'invention, celle-ci se rapporte aux composés cités ci-dessous ou à leurs ses sels physiologiquement acceptables pour leur utilisation pour la prévention et/ou le traitement de maladies et/ou désordres résultant d'un excès de NO :

- La quinone du verbascoside, présente sous 2 formes :

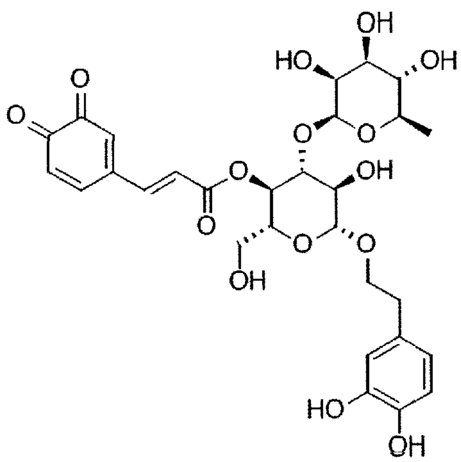

Première forme de la quinone du verbascoside

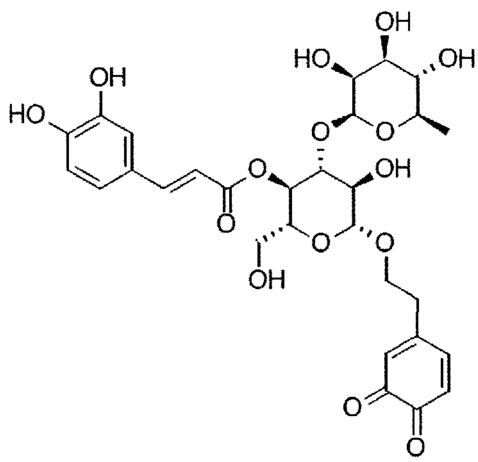

Deuxième forme de la quinone du verbascoside

Par chaîne alkyle en $C_1$-$C_3$ linéaire, éventuellement substituée, on désigne une chaîne hydrocarbonée de 1 à 3 atomes de carbone, saturée, linéaire, éventuellement substituée, telle que le méthyle, l'éthyle ou le propyle.
Par fonction hydroxyle, on désigne le groupement -OH.
Par fonction carboxylique, on désigne le groupement -COOH.

Par cycle benzénique, on désigne le groupe fonctionnel aromatique de formule brute $C_6H_6$ :

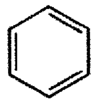.

Par diphénol, on désigne un composé aromatique, constitué d'un cycle benzénique et de deux fonctions hydroxyle, de formule brute $C_6H_6O_2$.

Par radical cafféoyl, on désigne le radical dérivé de l'acide caféique, de formule : -O-CO-CH=CH-(3,4)diphénol.

Par cycle pyranose, on désigne un cycle en $C_6$, saturé, constitué de 5 atomes de carbone et un atome d'oxygène.

A titre d'exemple, on peut citer le glucose qui possède un cycle pyranose, subsitué par un $-CH_2-OH$ et 4 hydroxyles -OH.

Par rhamnose, on désigne l'ose sous sa conformation D ou L, sous sa forme α ou β, de formule :

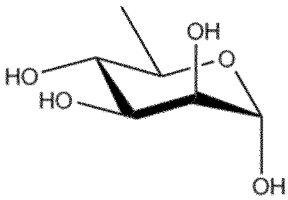 (α-D-rhamnose) ou 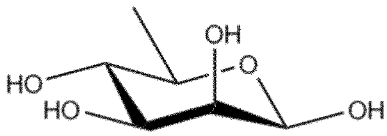 (β-D-rhamnose)

ou

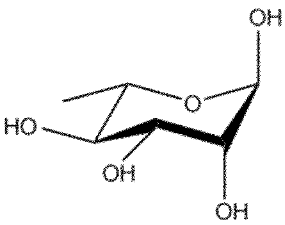 (α-L-rhamnose) ou 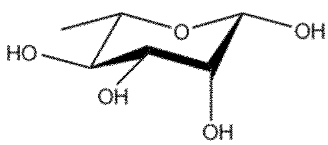 (β-L-rhamnose).

Les composés de formule (I) peuvent être synthétisés chimiquement ou bien extraits à partir de plantes.

Comme exemple, on peut citer l'orthoquinone de l'acide caféique qui peut être obtenue cristallisée, par oxydation de l'acide caféique par l'o-chloranil, selon une méthode décrite dans l'art antérieur (20, 21).

Alternativement, l'orthoquinone de l'acide caféique peut être extraite à partir de plantes telles que Salvia officinalis, Mentha spicata, Cinnamomum verum, Thymus vulgaris.

L'homme du métier connaît les méthodes d'extraction de composés chimiques à partir de plantes. Parmi ces méthodes, on peut citer la macération de feuilles de plantes dans de l'eau (25), l'extraction à l'aide de solvants chimiques, avec un fluide supercritique tel que le $CO_2$, la nanofiltration.

La quinone du verbascoside peut être extraite à partir de plantes de la famille des Lamiaceae (Phlomis, Scrophulariaceae, Verbascum phlomoides, Verbascum mallophorum), ou de la famille des Buddlejaceae (Buddleja globosa , Buddleja cordata), ou de la famille des Bignoniaceae (Pithecoctenium sp, Tynanthus panurensis), ou de la faimme des Orobanchaceae (Cistanche sp, Orobanche rapum-genistae), dans la famille des Plantaginaceae (Plantago lanceolata, Verbenaceae, Verbena officinalis, Aloysia citrodora) dans la famille des Oleaceae (Olea europaea) dans la famille des Lentibulariaceae (Pinguicula lusitanica) et dans la famille des Byblidaceae (Byblis liniflora).

En outre, les Inventeurs ont mis en évidence, de façon surprenante et contraire à ce qui était supposé dans l'art antérieur (19), qu'un extrait d'une plante connue pour son effet anti-inflammatoire, le plantain lancéolé (*Plantago lanceolata*), ne doit pas son effet anti-inflammatoire au glycoside phénylhydantoïque, le verbascoside qu'elle contient, mais à la quinone dérivant de ce polyphénol.

Ainsi, selon une autre variante de l'invention, celle-ci se rapporte à un extrait de *Plantago lanceolata* enrichi sélectivement en quinone de verbascoside ; un tel extrait de *Plantago lanceolata* enrichi en quinone de verbascoside est de préférence obtenu à partir de parties aériennes de la plante par macération alcoolique ; un tel extrait peut être obtenu selon le procédé décrit à l'exemple 5. Cet extrait particulier produit un effet significatif de neutralisation du NO.

**[0046]** L'invention se rapporte plus particulièrement à l'utilisation d'un composé ou d'un extrait de *Plantago lanceolata* selon l'invention pour la prévention et/ou le traitement de maladies et/ou désordres inflammatoires.

En particulier, les composés ou l'extrait de *Plantago lanceolata* selon l'invention sont utiles pour la prévention et/ou le traitement des maladies et/ou désordres inflammatoires cutanés tels que le psoriasis, la dermatite atopique, la dermatite de contact, l'irritation cutanée, la réaction d'hypersensibilité de contact, les manifestations allergiques cutanées, la vasodilatation excessive, la rosacée, l'érythème solaire, l'acné.

Les composés ou l'extrait de *Plantago lanceolata* selon l'invention sont encore utiles pour la prévention et/ou le traitement des maladies et/ou désordres inflammatoires articulaires telles que l'arthrite, comprenant l'arthrite rhumatoïde, l'arthrite infectieuse et l'osteoarthrite, l'arthrose, la polyarthrite rheumatoïde, la spondylarthrite ankylosante, le lupus erythémateux, la chondrite.

Des auteurs ont montré que des extraits de *Plantago lanceolata* permettaient une réduction du nombre de quintes de toux chez des cobayes (24). Les composés ou l'extrait de *Plantago lanceolata* selon l'invention peuvent être utilisés pour prévenir ou traiter la toux, qu'elle soit associée à une maladie des voies respiratoires telles que la bronchiolite du nourrisson, la bronchite, le rhume, la coqueluche, la tuberculose, un cancer bronchique ou associée à un état et/ou un environnement particulier comme c'est le cas de la toux du fumeur ou provoquée par le tabagisme passif, la toux allergique, la toux associée à de l'asthme ou encore à un reflux gastro-oesophagien.

Les composés ou l'extrait de *Plantago lanceolata* selon l'invention peuvent encore être utilisés pour prévenir ou traiter des collapsus cardio-vasculaires associés à un choc hypovolémique, ou à un choc cardiogénique ou à un choc anaphylactique ou à un choc septique. Le collapsus cardio-vasculaire correspond à un effondrement de la pression sanguine ; la pression artérielle systolique devient alors inférieure à 80 mmHg.

L'utilisation des composés ou de l'extrait de *Plantago lanceolata* selon l'invention peut également avoir une finalité cosmétique, afin de lutter contre le vieillissement intrinsèque ou extrinsèque de la peau, plus particulièrement pour lutter contre les signes du vieillissement cutané, tels que le photovieillissement, les rides, les ridules, les peaux sèches ou craquelées.

Parmi les autres utilisations de ces composés ou de l'extrait de *Plantago lanceolata* selon l'invention en cosmétique, on peut citer :

- la lutte contre les processus inflammatoires neurogéniques cutanés ;
- l'amélioration du confort des peaux sensibles ;
- le renforcement de la fonction barrière de la peau ;
- la stimulation de l'hydratation de la peau ;
- l'amélioration du confort des peaux sèches ;
- le contrôle de la sudation ;
- la stimulation de la lipolyse ;
- l'inhibition de la chute des cheveux.

La présente invention se rapporte également à une composition pharmaceutique ou cosmétique contenant au moins un composé de formule (I) ou un extrait de *Plantago lanceolata* selon l'invention ainsi qu'un véhicule physiologiquement acceptable.

L'homme du métier saura adapter la formulation des compositions selon l'invention en fonction de leurs propriétés physico-chimiques et leur voie d'administration.

Les compositions selon l'invention pourront être administrées par toute voie d'administration qui inclut notamment la voie topique, en particulier sur la peau ou les cheveux, mais également la voie orale.

De préférence, lorsque les compositions selon l'invention sont utilisées pour le traitement de maladies et/ou de désordres inflammatoires cutanés ou articulaires, ou bien lorsqu'elles sont utilisées pour une finalité cosmétique, elles sont administrées par la voie topique et peuvent être choisies parmi une crème, un gel, une huile, une lotion, un lait.

Lorsque les compositions selon l'invention sont utilisées pour traiter la toux, on préfèrera les administrer par voie orale. La forme galénique choisie sera un sirop, une pastille à sucer, une gélule, un comprimé.

**FIGURE** :

Figure 1 : Appareil permettant de mettre en œuvre la méthode spécifique d'évaluation de la neutralisation d'oxyde nitrique telle que décrite dans l'exemple 1.

## EXEMPLES

### Exemple 1. Méthode d'évaluation de la neutralisation de l'oxyde nitrique

L'oxyde nitrique est préparé extemporanément dans un récipient A, obturé par un bouchon souple en polymère à travers lequel passent une tubulure entrante et une tubulure sortante, d'un diamètre intérieur compris entre 50 μm et

300 µm, mais particulièrement de 100 µm (cf. figure 1), d'un volume compris entre 10 ml et 100 ml, mais particulièrement de 30 ml, en ajoutant un volume compris entre 0,1 et 2 ml, mais particulièrement de 0,5 ml d'une solution aqueuse de nitrite de sodium ($NaNO_2$, réf. Sigma-Aldrich 237213) à une concentration comprise entre 0,10 % et 2,00 %, mais particulièrement de 0,70 %, à un volume compris entre 5 ml et 80 ml de la solution aqueuse suivante :

| | |
|---|---|
| Acide sulfurique ($H_2SO_4$, réf. Sigma-Aldrich 32,050-1) : | 1 ml |
| Sulfate ferreux ($FeSO_4$, $7H_2O$, réf. Sigma-Aldrich 215422) : | 5,5 g |
| Eau désionisée : | 200 ml |

Le récipient B dans lequel est mesuré l'oxyde nitrique est un récipient opaque à la lumière, à double enveloppe permettant une circulation d'eau maintenant la température à 25°C, obturé d'un bouchon à travers lequel passent la tubulure provenant du récipient A et une tubulure d'échappement (cf. figure 1), d'un volume compris entre 10 ml et 100 ml, de préférence de 30 ml qui reçoit un volume compris entre 5 ml et 80 ml, de préférence de 20 ml, d'une solution aqueuse de tampon phosphate pH 7,4.

Une fois mélangées les solutions sont agitées pendant toute la durée de la mesure par un agitateur magnétique, dans le récipient A et le récipient B.

De l'azote gazeux est admis à un débit compris entre 20 ml/min et 120 ml/min, de préférence de 80 ml/min dans le fond du récipient A. L'azote entraine ainsi l'oxyde nitrique par la tubulure sortante du récipient A jusque dans le récipient B où il sera mesuré par un dispositif prévu à cet effet équipé d'une sonde ampérométrique pourvue d'une membrane spécifique (appareil de réf. TBR 1025 de la société World Précision Instruments, sonde ISO-NOP). La sonde délivre ainsi un courant variable entre 10 et 1000 nA proportionnel à la concentration d'oxyde nitrique dans la solution testée, de l'ordre de grandeur de la µM. Le courant électrique produit par la sonde sous l'influence de l'oxyde nitrique est enregistré par un dispositif approprié.

Avant toute mesure on étalonne la sonde en mesurant le courant produit par la libération de quantités connues croissantes d'oxyde nitrique. On vérifie ainsi qu'il existe une relation linéaire entre le courant électrique produit par la sonde et la concentration molaire d'oxyde nitrique et on détermine la correspondance entre ce courant électrique et la concentration molaire d'oxyde nitrique. Pour cela, on place 20 ml de la solution aqueuse suivante dans le récipient B déconnecté du récipient A pendant la durée de l'étalonnage :

| | |
|---|---|
| Acide sulfurique ($H_2SO_4$, réf. Sigma-Aldrich 32,050-1) : | 1 ml |
| Iodure de potassium (KI, réf. Sigma-Aldrich 12636) : | 3,31 g |
| Eau désionisée : | 200 ml |

On ajoute ensuite successivement 50 µl, 100 µl, 150 µl et 200 µl d'une solution de nitrite de sodium ($NaNO_2$, réf. Sigma-Aldrich 237213) à une concentration de 360 µM.

On trace la courbe faisant correspondre la concentration en oxyde nitrique dans le milieu réactionnel et l'intensité électrique produite par la sonde exprimée en nA pour chaque addition de la solution d'oxyde nitrique.

Evaluation de l'effet neutralisant d'une molécule à tester

Lors d'une mesure typique, on place 20 ml de la solution de sulfate ferreux dans le récipient A et 20 ml de tampon phosphate pH 7,4 dans le récipient B. Dans le récipient A, on admet de l'azote gazeux pendant 10 min, qui transite vers le récipient B, afin d'éliminer l'oxygène dissous présent dans les solutions des récipients A et B. Après ces 10 min, on lance l'enregistrement de la mesure de l'oxyde nitrique.

Au temps $T_0$, on injecte la solution de nitrite de sodium dans le récipient A à travers le bouchon de polymère souple grâce à une seringue équipée d'un aiguille. L'enregistrement de l'oxyde nitrique montre une évolution positive qui va se stabiliser brièvement après environ 3 min, avant de diminuer lentement pour revenir à la ligne de base en environ 20 min. Au temps $T_1 = T_0 + 4$ min, on injecte 400 µl de solution hydro-éthanolique à un titre alcoolique compris entre 0 % et 100 % selon la solubilité du produit à tester, contenant une quantité P de produit de 4 à 8 mg, selon l'efficacité du produit à tester, à travers le bouchon souple du récipient B grâce à une seringue équipée d'une aiguille.

On réalise un témoin avec 400 µl de solvant dans lequel est dissout le produit à tester.

La valeur E de l'effet de neutralisation de l'oxyde nitrique par le produit à tester, exprimée en µM d'oxyde nitrique neutralisé par seconde et par mg de produit à tester est obtenue par l'expression suivante :

$$(1) \qquad E = \frac{\Delta H_{test} - \Delta H_{tem}}{30P}$$

Avec : $\Delta H_{test}$ = différence de concentration en oxyde nitrique essai, exprimée en pM d'oxyde nitrique entre $T_1$ et $T_2 = T_1$ + 30 sec, obtenue avec la solution de produit à tester.
$\Delta H_{tem}$= différence de concentration en oxyde nitrique témoin, exprimée en pM d'oxyde nitrique entre $T_1$ et $T_2 = T_1$ + 30 sec, obtenue avec le solvant du produit à tester.
P = quantité de produit à tester en mg.

**Exemple 2. Mesure de l'effet de neutralisation de l'oxyde nitrique et effets de diverses molécules**

Le test décrit dans l'exemple 1 a été mis en œuvre pour tester les molécules suivantes :

Acide rosmarinique (Aldrich 536954)
Acide p-coumarique (Sigma C9008)
(+) Catechin hydrate (Sigma C1251)
Acide caféique (Sigma-Aldrich C0625)
Acide chlorogénique (Sigma-Aldrich C3878)
Quercetin dihydrate (Sigma Q0125)
Hémoglobine porcine (Sigma H4131)
Verbascoside (HWI Analytik GMGH 0082-05-80)
Lutéoline (Sigma L9283)

Après avoir étalonné la sonde de mesure de la concentration de l'oxyde nitrique de l'appareillage décrit à l'exemple 1, dans le récipient A, tel que décrit à l'exemple 1 et illustré à la figure 1, on place 20 ml une solution aqueuse de :

| | |
|---|---|
| Acide sulfurique ($H_2SO_4$, réf. Sigma-Aldrich 32,050-1) : | 1 ml |
| Sulfate ferreux ($FeSO_4$, $7H_2O$, réf. Sigma-Aldrich 215422) : | 5,5 g |
| Eau désionisée : | 200 ml |

Dans le récipient B, tel que décrit ci-dessus et figure 1, on place 20 ml de solution tampon phosphate à pH 7,4.
On admet de l'azote gazeux dans l'appareil à un débit de 80 ml/min pendant 10 min, puis on lance l'enregistrement de la valeur de l'intensité de courant électrique produit par la sonde de détection de l'oxyde nitrique et on injecte (au temps $T_0$) dans le récipient A, 0,5 ml d'une solution aqueuse de nitrite de sodium ($NaNO_2$, ref. Sigma-Aldrich 237213) à une concentration de 0,70 %.
Au temps $T_1 = T_0$+ 4 min, on injecte 400 µl de solution hydro-éthanolique à un titre alcoolique compris entre 0 % et 100 % selon la solubilité du produit à tester, contenant une quantité P de produit à tester exprimée en mg, ajustée selon l'efficacité du produit testé.
On note les valeurs des concentrations en oxyde nitrique au temps $T_0$, $T_1 = T_0$ + 4 min et $T_2 = T_1$+ 30 sec. Les valeurs de E calculées selon la formule (1) figure dans le tableau 1 ci-dessous.

Tableau 1.

| **Molécules testées** | **E (µM.s$^{-1}$.mg$^{-1}$)** |
|---|---|
| Hémoglobine (contrôle +) | 2,27 |
| Acide caféique | 0,957 |
| Acide rosmarinique | 0,043 |
| Acide p-coumarique | 0,13 |
| Catéchine | 0,13 |
| Acide chlorogénique | 0,043 |
| Lutéoline | 0,174 |
| Quercétine | 0,087 |
| Verbascoside | 0,058 |

**Exemple 3. Préparation et mesure de l'effet de l'o-quinone de l'acide caféique** (exemple de référence)

L'orthoquinone de l'acide caféique :

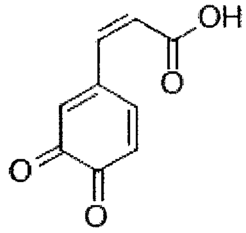

Caffeoyl-quinone

peut être obtenue cristallisée, par oxydation de l'acide caféique par l'o-chloranil en solution dans un mélange d'éther et de tétrahydrofurane (4/1) à -70°C, selon une méthode décrite dans l'art antérieur (20, 21).
Selon la méthode de mesure de l'effet de neutralisation de l'oxyde nitrique telle que décrite dans l'exemple 1, la caffeoylquinone produit un effet E de 24,36 $\mu M.s^{-1}.mg^{-1}$.
On observe donc une valeur élevée de E pour cette molécule comparativement aux effets produits par des molécules déjà connues comme capables de neutraliser l'oxyde nitrique.

**Exemple 4. Préparation d'un extrait de plantain et évaluation de son activité de neutralisation de l'oxyde nitrique.**

Afin de déterminer la méthode de traitement de la plante et la méthode d'extraction conduisant à la meilleure efficacité de neutralisation de l'oxyde nitrique, il a été procédé à l'extraction de plante sèche et de plante fraîche par des solutions hydroalcooliques de titres variant de 10 en 10 allant de 0 % à 100 % d'éthanol, soit par macération à température ambiante pendant 48h, dans des volumes de solvant correspondant à 10 fois le poids de la plante (v/p), soit par extraction à ébullition à reflux pendant 30 min par des volumes de solvants correspondant également à 10 fois le poids de la plante (v/p). A la suite de ces essais préliminaires, la méthode suivante a été choisie, car elle produit l'extrait le plus actif :
Des parties aériennes de *Plantago lanceolata* sont récoltées et rapidement mises en congélation à -18°C afin d'assurer leur conservation. 1 kg de ce lot est broyé grossièrement et mis à macérer dans 10 litres d'éthanol aqueux à 60 % pendant 48 h. Ce titre alcoolique est défini en tenant compte de la teneur en eau de la plante, préalablement déterminée par dessiccation sur un échantillon représentatif. A la fin de la macération la plante est éliminée par tamisage, puis la solution extractive est filtrée sur un filtre de 0,45 µm de porosité pour éliminer la plus grande partie des germes présents. La solution est ensuite concentrée sous pression réduite de façon à obtenir un volume total de 11, totalement dépourvu d'éthanol.
Cette solution est ensuite séchée par lyophilisation pour obtenir un poids final de 43 g.
L'efficacité E de neutralisation de l'oxyde nitrique est de 0,16 $\mu M.s^{-1}.mg^{-1}$.

**Exemple 5. Préparation d'un extrait de parties aériennes de *Plantago lanceolata* enrichi en o-quinones issues du verbascoside**

Des parties aériennes de *Plantago lanceolata* sont récoltées et rapidement mises en congélation à -18°C afin d'assurer leur conservation. 1 kg de ce lot est broyé grossièrement et mis à macérer dans 10 litres d'éthanol aqueux à 60 % pendant 48 h. Ce titre alcoolique est défini en tenant compte de la teneur en eau de la plante, préalablement déterminée par dessiccation sur un échantillon représentatif. A la fin de la macération, la plante est éliminée par tamisage, puis la solution extractive est filtrée sur un filtre de 0,45 µm de porosité pour éliminer la plus grande partie des germes présents. La solution est ensuite concentrée sous pression réduite de façon à obtenir un volume total de 11, totalement dépourvu d'éthanol.
Cette solution concentrée est ensuite extraite à contre courant par 5 fois 200 ml d'acétate d'éthyle. Les solutions organiques sont réunies, séchées sur sulfate de sodium anhydre, filtrées, puis évaporées à sec sous pression réduite. On obtient ainsi 1,12 g d'extrait sec qui est repris par 25 ml d'éthanol pur.
Cette solution éthanolique est soumise à une flash chromatographie (appareillage Grace Reveleris X2) sur une colonne de gel de silice de 40 g (Grace Reveleris Silica Cartridge 40 g) par un gradient dichlorométhane-méthanol comme dans le tableau 2 ci-dessous:

Tableau 2

| Temps (mn) | Dichlorométhane % | Méthanol % |
|---|---|---|
| 0 | 100 | 0 |
| 15 | 80 | 20 |
| 18 | 80 | 20 |
| 18,5 | 50 | 50 |
| 23,5 | 50 | 50 |

On recueille des fractions de 2 ml qui sont soumises au test de neutralisation de l'oxyde nitrique. On conserve les fractions les plus actives qui correspondent aux volumes d'élution de 568 ml à 576 ml, qui fournissent ensemble 17,7 mg de matière sèche après évaporation du solvant sous pression réduite, et qui possède une efficacité E de neutralisation de l'oxyde nitrique de 0,96 $\mu M.s^{-1}.mg^{-1}$.

On vérifie ensuite la présence de l'o-quinone du verbascoside en spectrométrie de masse haute résolution en tandem, avec ionisation électrospray en mode négatif, par la présence d'un ion doublement chargé à m/z 311, correspondant à la quinone considérée et présentant la fragmentation caractéristique des hétérosides phenylhydantoïques, dans ce cas apparentée à celle du verbascoside.

**Exemple 6. Formule galénique d'une crème contenant un extrait de plantain, destinée à traiter les inflammations cutanées (notamment dermatites, eczéma, psoriasis) adaptée à l'usage corporel.**

On prépare une crème possédant la formule pondérale suivante, selon un procédé et avec du matériel standards tel que connu de l'art antérieur :

| | |
|---|---|
| Eau purifiée : | 67,28 % |
| Glycérol (Glycérine, AMI Chimie) : | 20 % |
| Alcool cétostéarylique (Lanette O, AMI Chimie) : | 7 % |
| Acide palmitostéarique (Stéarine, Stéarinerie Dubois) : | 3 % |
| Extrait de parties aériennes fraîches de *Plantago lanceolata* tel que décrit dans l'exemple 5 : | 1 % |
| Alcool benzylique (Geogard 221, Lonza) : | 0,87 % |
| Sulfate sodique cétostéarylique (Lanette E, AMI Chimie) : | 0,7 % |
| Acide déhydroacétique (Geogard 221, Lonza) : | 0,09 % |
| Hydroxyde de sodium (Sigma-Aldrich) : | 0,058 % |

**Exemple 7. Formule galénique d'une crème contenant un extrait de plantain, destinée à traiter les inflammations cutanées (notamment dermatites, eczéma, psoriasis) adaptée à l'usage sur le visage.**

| | |
|---|---|
| Eau purifiée : | 67,28 % |
| Glycérol (Glycérine, AMI Chimie) : | 10 % |
| Alcool cétostéarylique (Lanette O, AMI Chimie) : | 7 % |
| Myristate d'isopropyle (Isopropyle Myristate, Interchimie) : Extrait de parties aériennes fraîches de *Plantago lanceolata* tel que décrit dans l'exemple 5 : | 3 % 1 % |
| Alcool benzylique (Geogard, Lonza) : | 0,87 % |
| Sulfate sodique cétostéarylique (Lanette E, AMI Chimie) : | 0,7 % |
| Acide déhydroacétique (Geogard, Lonza) : | 0,09 % |
| Hydroxyde de sodium (Sigma-Aldrich) : | 0,058 % |

**Exemple 8. Formule galénique d'un gel lavant contenant un extrait de plantain, destinée au nettoyage des cheveux, du visage et du corps de sujets présentant des inflammations cutanées (notamment dermatites, eczéma, psoriasis)**

| | |
|---|---|
| Mélange d'éthersulfates d'alcool gras (Texapon ASV50, AMI Chimie) : | 50 % |
| Eau purifiée : | 34,03 % |
| Glycérol (Glycérine, AMI Chimie) : | 10 % |
| Chlorure de sodium (sodium chloride VWR) : | 4 % |
| Extrait de parties aériennes fraîches de *Plantago lanceolata* tel que décrit dans l'exemple 5 : | 1 % |
| Alcool benzylique (Geogard 221, Lonza) : | 0,87 % |
| Acide déhydroacétique (Geogard 221, Lonza) : | 0,09 % |
| Hydroxyde de sodium (Sigma-Aldrich) : | 0,01 % |

**Exemple 9. Formule galénique d'un gel destiné à traiter les inflammations cutanées (dermatites, eczéma, psoriasis) adaptée à l'usage corporel et sur le visage.**

| | |
|---|---|
| Eau purifiée : | 95,69 % |
| Carbomer (Carbopol 980, Lubrizol) : | 2 % |
| Extrait de parties aériennes fraîches de *Plantago lanceolata* tel que décrit dans l'exemple 5 : | 1 % |
| Alcool benzylique (Geogard 221, Lonza) : | 0,87 % |
| Acide déhydroacétique (Geogard 221, Lonza) : | 0,09 % |
| Hydroxyde de sodium (Sigma-Aldrich) : | 0,35 % |

**Exemple 10. Formule galénique d'une crème contenant un extrait de plantain, destinée à traiter les inflammations relatives à l'acné.**

| | |
|---|---|
| Eau purifiée : | 71,04 % |
| Alcool cétostéarylique (Lanette O, AMI Chimie) : | 10 % |
| Huile essentielle de citron (Myrtéa) : | 7,5 % |
| Huile essentielle d'orange douce (Myrtéa) : | 7,5 % |
| Extrait de parties aériennes fraîches de *Plantago lanceolata* tel que décrit dans l'exemple 5 : | 1 % |
| Sulfate sodique cétostéarylique (Lanette E, AMI Chimie) : | 2 % |
| Alcool benzylique (Geogard 221, Lonza) : | 0,87 % |
| Acide déhydroacétique (Geogard 221, Lonza) : | 0,09 % |

**Exemple 11. Formule galénique d'un sirop pour la toux**

| | |
|---|---|
| Eau purifiée : | 44,23 % |
| Saccharose : | 44,22 % |
| Glycérol (Glycérine, AMI Chimie) : | 5% |
| Extrait de parties aériennes fraîches de *Plantago lanceolata* tel que décrit dans l'exemple 5 : | 4% |
| Arôme naturel de framboise : | 1,5 % |
| Acide citrique monohydraté : | 0,75 % |
| Benzoate de sodium : | 0,3 % |

**Exemple 12. Formule galénique de gélules pour traiter les manifestations inflammatoires de l'arthrose.**

Pour une gélule de gélatine n°0 (Capsugel) :

| | |
|---|---|
| Extrait de parties aériennes fraîches de *Plantago lanceolata* tel que décrit dans l'exemple 5 : | 0,350 g |
| Maltodextrine : | 0,045 g |

REFERENCES

1. Vascular endothelial cells synthetize nitric oxide from L-arginine. R.M. Palmer, D.S. Ashton, S. Moncada. Nature, Vol. 333, 6174: 664-666.
2. Nitric oxide release accounts for the biological activity of endothelium-derived relaxing factor. R.M. Palmer, A.G. Ferrige, S. Moncada. Nature, Vol. 327, 6122: 524-526.
3. Macrophage Oxidation of L-Arginine to Nitrite and Nitrate: Nitric Oxide Is an Intermediate. M. A. Marietta, P. S. Yoon, R. Iyengar, C. D. Leaf, J. S. Wishnok. Biochemistry, 1988, 27: 8706-8711.
4. Nitric oxide: a cytotoxic activated macrophage effector molecule. J.B. Hibbs, R.R. Taintor, Z. Vavrin, E.M. Rachlin. Biochemical and Biophysical Communications, 1988, Vol. 157, 1: 87-94.
5. Biosynthesis of nitric oxide from L-Arginine. A pathway for the regulation of cell function and communication. S. Moncada, R.M.J. Palmer, E.A. Higgs. Biochemical Pharmacology, 1989, Vol. 38, 11: 1709-1715.
6. Modulation of acute inflammation by endogenous nitric oxide. A. Ialenti, A. Ianaro, S. Moncada, M. Di Rosa. European Journal of Pharmacology, 1992, Vol 211: 177-182.
7. Nitric oxide and its implication in skin homeostasis and disease - A review. D. Bruch-Gerharz, T. Ruzicka, V. Kolb-Bachofen. Arch. Dermatol. Res., 1998,290: 643-651.
8. Nitric Oxide Levels in Patients with Psoriasis Treated with Methotrexate. N. S. Tekin, N. Ilter, B. Sancak, M. G. Ozden, M. A. Gurer. Mediators of Inflammation, 2006, Vol. 2006: 1-5.
9. New markers of disease activity in children with atopic dermatitis. A. H. A. Mohsen, H. A. Wahab, E. Allam. Journal of American Science, 2011, Vol. 7(10): 404-408.
10. Nitric oxide function in the skin. M.-M. Cals-Grierson, A.D. Ormerod. Nitric Oxide, 2004, Vol. 10: 179-193.
11. The role of nitric oxide in sepsis - an overview. K. A. Kirkeboen, A. Strand. Acta Anaesthesiol. Scand., 1999, Vol. 43: 275-288.
12. Role of Nitric Oxide in Anaphylactic Shock. H. Mitsuhata, R. Shimizu, M. M. Yokoyama. Journal of Clinical Immunology, 1995, Vol. 15 (6).
13. Arginine-Based Inhibitors of Nitric Oxide Synthase: Therapeutic Potential and Challenges. J. VÍteček, A. Lojek, G. Valacchi, L. Kubala. Mediators of Inflammation, 2012, Vol. 2012 : 1-22.
14. Inhibition of nitric oxide synthase during sepsis: revival because of isoform selectivity, W. Stahl, M. Matejovic, P. Radermacher. Shock, 2010, Vol. 34, (3): 321-322.
15. Nos inhibitors for treatment of wrinkles. S. Fujii, E. Lerner. Brevet PCT/US2002/002292.
16. Inhibiteurs de NO synthases et utilisation. M.M. Cals-Grierson. Brevet PCT/FR2002/002064.
17. Beneficial effect of carboxy-PTIO on hemodynamic and blood gas changes in septic shock dogs. Chieko Mitaka, Yukio Hirata, Kuninori Yokoyama, Takashi Nagura, Yukio Tsunoda and Keisuke Amaha. Crit Care 1997, 1:45.
18. Ruthenium complexes as nitric oxide scavengers: a potential therapeutic approach to nitric oxide-mediated diseases. Fricker SP, Slade E, Powell NA, Vaughan OJ, Henderson GR, Murrer BA, Megson IL, Bisland SK, Flitney FW. Br. J. Pharmacol. 1997, 122(7):1441-9.
19. Assessment report on *Plantago lanceolata* L., folium. Collective work. European Medicines Agency. EMA/HMPC/437859/2010. Committee on Herbal Medicinal Products (HMPC).
20. The synthesis and isolation of caffeoquinone and caffeoquinone methyl ester. Davies R. Tetrahedron letters, 1976, 4: 313-314.
21. Oxidation Products of Caffeic Acid as Model Substances for the Antigonadotropic Activity of Plant Extracts. Mathias John, Hans Gerd Gumbinger, and Hilke Winterhoff. Planta Medica, 1990, 56:14-18.
22. Nitric oxide in arthritis. Daniel Jang and George A. C. Murrell. Free Radical Biology & Medicine, 1998, 24 (9) : 1511-1519.
23. WO 02/07720
24. Antitussive effect of Plantigo lanceolate in Guinea Pigs. Boskabady M.H, Rakhshandah H., Afiat M, Aelami Z, Amiri S. Iran J Med Sci. 2006, 31(3): 143-146.
25. FR 2 733 419.

## Revendications

**1.** Composé de formule (I) :

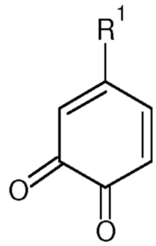

(I)

dans laquelle $R^1$ est sélectionné parmi le groupe constitué de :

- $-CH=CH-COOR^2$

  avec $R^2$ représente la formule -Y-O-X-(3,4)diphénol (II) avec :

  • Y représente un cycle pyranose, substitué par un $CH_2$-OH et au moins une fonction hydroxyle et substitué par un rhamnose ;
  • X représente la chaîne $-CH_2-CH_2-$ ;

- $-Z-O-R^3$

  Avec :

  - Z représente la chaîne $-CH_2-CH_2-$;
  - $R^3$ représente la formule -W-O-CO-CH=CH-(3,4)diphénol (III) avec W représente un cycle pyranose, substitué par un $CH_2$-OH et au moins une fonction hydroxyle et substitué par un rhamnose ;

  pour son utilisation pour la prévention et/ou le traitement de maladies et/ou désordres inflammatoires.

**2.** Composé pour son utilisation selon la revendication 1 choisi parmi la 1ère forme de la quinone du verbascoside et la 2ème forme de la quinone du verbascoside.

**3.** Composé pour son utilisation selon la revendication 2, **caractérisé en ce qu'**il est contenu dans un extrait de *Plantago lanceolata.*

**4.** Composés selon l'une des revendications 1 à 3, pour leur utilisation pour la prévention et/ou le traitement de maladies et/ou désordres inflammatoires cutanés choisis parmi le psoriasis, la dermatite atopique, la dermatite de contact, l'irritation cutanée, la réaction d'hypersensibilité de contact, les manifestations allergiques cutanées, la vasodilatation excessive, la rosacée, l'érythème solaire, l'acné.

**5.** Composés selon l'une des revendications 1 à 3, pour leur utilisation pour la prévention et/ou le traitement de maladies et/ou désordres inflammatoires articulaires choisis parmi l'arthrite, l'arthrose, la polyarthrite rhumatoïde, la spondylarthrite ankylosante, le lupus érythémateux, la chondrite.

**6.** Composés selon l'une des revendications 1 à 3, pour leur utilisation pour la prévention et/ou le traitement de la toux.

**7.** Composés selon l'une des revendications 1 à 3, pour leur utilisation pour la prévention et/ou le traitement des chocs anaphylactiques ou des chocs septiques.

**8.** Composés selon l'une des revendications 1 à 3, pour leur utilisation pour lutter contre le vieillissement intrinsèque

ou extrinsèque de la peau ; pour lutter contre les signes du vieillissement cutané, tels que le photovieillissement, les rides, les ridules, les peaux sèches ou craquelées ; pour lutter contre les processus inflammatoires neurogéniques cutanés ; pour améliorer le confort des peaux sensibles ; pour renforcer la fonction barrière de la peau ; pour stimuler l'hydratation de la peau ; pour améliorer le confort des peaux sèches ; pour contrôler la sudation ; pour stimuler la lipolyse ; pour inhiber la chute des cheveux.

**9.** Composition pharmaceutique ou cosmétique contenant au moins un composé de formule (I) tel que décrit dans l'une des revendications 1 à 3 ainsi qu'un véhicule physiologiquement acceptable.

**10.** Composition selon la revendication 9, **caractérisée en ce qu'**elle est destinée à être appliquée sur la peau ou à être administrée oralement.

**11.** Composition selon la revendication 10, **caractérisée en ce qu'**elle est une crème, un gel, une huile, une lotion, un lait.

**12.** Composition selon la revendication 10, **caractérisée en ce qu'**elle est un sirop, une pastille à sucer, une gélule, un comprimé.

**Patentansprüche**

**1.** Verbindung der Formel (I):

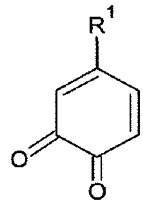

(I)

in der $R^1$ aus der Gruppe ausgewählt wird, die gebildet ist aus:

•

-CH=CH-COOR$^2$

wobei $R^2$ die Formel -Y-O-X-(3,4)Diphenol (II) repräsentiert, mit:

• Y, das einen Pyranosezyklus repräsentiert, der durch $CH_2$-OH und mindestens eine Hydroxylfunktion substituiert ist und substituiert durch eine Rhamnose;
• X die Kette $-CH_2-CH_2-$ repräsentiert;

•

-Z-O-$R^3$

Mit:

- Z, das die-$CH_2$-$CH_2$- Kette repräsentiert;
- $R^3$, das die Formel -W-O-CO-CH=CH-(3,4)Diphenol (III) repräsentiert, wobei W einen Pyranosezyklus repräsentiert, der durch ein $CH_2$-OH und mindestens eine Hydroxylfunktion substituiert ist und substituiert durch eine Rhamnose;
für deren Verwendung bei der Vorbeugung und/oder Behandlung von entzündlichen Krankheiten und/ oder Störungen.

**2.** Verbindung für deren Verwendung nach Anspruch 1, ausgewählt aus der 1. Form des Verbascosid-Chinons und der 2. Form des Verbascosid-Chinons.

3. Verbindung für deren Verwendung nach Anspruch 2, **dadurch gekennzeichnet, dass** sie in einem Extrakt aus *Plantago lanceolata* enthalten ist.

4. Verbindungen nach einem der Ansprüche 1 bis 3, für deren Verwendung bei der Vorbeugung und/oder Behandlung von entzündlichen Hautkrankheiten und/ oder -störungen, ausgewählt aus Schuppenflechte, atopischem Ekzem, Kontaktdermatitis, Hautreizung, Überempfindlichkeitsreaktion auf Kontakt, allergischen Hautreaktionen, übermäßiger Blutgefäßerweiterung, Rosazea, Sonnenbrand, Akne.

5. Verbindungen nach einem der Ansprüche 1 bis 3, für deren Verwendung bei der Vorbeugung und/oder Behandlung von entzündlichen Gelenkskrankheiten und/ oder -störungen, ausgewählt aus Arthritis, Arthrose, rheumatoider Arthritis, Spondylitis ankylosans, Lupus erythermatodes, Chondrit.

6. Verbindungen nach einem der Ansprüche 1 bis 3, für deren Verwendung bei der Vorbeugung und/oder Behandlung von Husten.

7. Verbindungen nach einem der Ansprüche 1 bis 3, für deren Verwendung bei der Vorbeugung und/oder Behandlung von anaphylaktischen Schocks oder septischen Schocks.

8. Verbindungen nach einem der Ansprüche 1 bis 3, für deren Verwendung bei der Bekämpfung von intrinsischer oder extrinsischer Hautalterung; bei der Bekämpfung von Zeichen der Hautalterung, wie Alterung durch Lichteinwirkung, Falten, Runzeln, trockener oder rissiger Haut; bei der Bekämpfung der neurogenen Hautentzündungsprozesse; bei der Verbesserung des Komforts bei empfindlicher Haut; bei der Verstärkung der Sperrfunktion der Haut; beim Stimulieren der Hydratation der Haut; bei der Verbesserung des Komforts bei trockener Haut; bei der Kontrolle der Schweißabsonderung; beim Stimulieren der Lipolyse; beim Unterdrücken von Haarausfall.

9. Pharmazeutische oder kosmetische Zusammensetzung, mindestens eine Verbindung der Formel (I) nach einem der Ansprüche 1 bis 3, sowie einen physiologisch verträglichen Träger enthaltend.

10. Zusammensetzung nach Anspruch 9, **dadurch gekennzeichnet, dass** sie dazu bestimmt ist, auf die Haut aufgetragen oder oral verabreicht zu werden.

11. Zusammensetzung nach Anspruch 10, **dadurch gekennzeichnet, dass** sie eine Creme, ein Gel, ein Öl, eine Lotion oder eine Milch ist.

12. Zusammensetzung nach Anspruch 10, **dadurch gekennzeichnet, dass** sie ein Sirup, eine Lutschpastille, eine Kapsel oder eine Tablette ist.

## Claims

1. Compound of formula (I) :

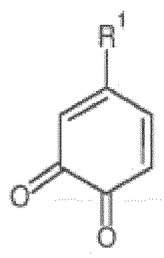

(I)

wherein $R^1$ is selected from the group consisting of:

- $-CH=CH-COOR^2$

with $R^2$ representing the formula -Y-O-X-(3,4) diphenol (II) with:

- Y representing a pyranose ring, substituted by a $CH_2$-OH and at least one hydroxyl function and substituted by a rhamnose;
- X representing the chain $-CH_2-CH_2-$;

•

-Z-O-$R^3$

With:

- Z representing the chain $-CH_2-CH_2-$;
- $R^3$ representing the formula -W-O-CO-CH=CH-(3,4) diphenol (III) with W representing a pyranose ring, substituted by a $CH_2$-OH and at least one hydroxyl function and substituted by a rhamnose;

for use in the prevention and/or the treatment of inflammatory diseases and/or disorders.

**2.** Compound for the use thereof according to claim 1 selected from the 1st form of quinone from verbascoside and the 2nd form of quinone from verbascoside.

**3.** Compound for the use thereof according to claim 2, **characterised in that** it is contained in a *Plantago lanceolata* extract.

**4.** Compound according to one of claims 1 to 3, for use in the prevention and/or the treatment of inflammatory skin diseases and/or disorders selected from psoriasis, atopic dermatitis, contact dermatitis, skin irritation, contact hypersensitivity reaction, skin allergy manifestations, excessive vasodilatation, rosacea, sunburn, acne.

**5.** Compounds according to one of claims 1 to 3, for use in the prevention and/or the treatment of inflammatory joint diseases and/or disorders selected from arthritis, arthrosis, rheumatoid polyarthritis, ankylosing spondylitis, lupus erythematosus, chondritis.

**6.** Compounds according to one of claims 1 to 3, for use in the prevention and/or the treatment of a cough.

**7.** Compounds according to one of claims 1 to 3, for use in the prevention and/or the treatment of anaphylactic shocks or septic shocks.

**8.** Compounds according to one of claims 1 to 3, for use in the prevention and/or the treatment of intrinsic or extrinsic ageing of the skin; to fight against signs of skin ageing, such as photoaging, wrinkles, fine lines, dry or cracked skin; to fight against inflammatory skin neurogenic processes; to improve the comfort of sensitive skin; to strengthen the barrier function of the skin; to stimulate the hydration of the skin; to improve the comfort of dry skin; to control sweating; to stimulate lipolysis; to suppress hair loss.

**9.** Pharmaceutical or cosmetic composition containing at least one compound of formula (I) such as described in one of claims 1 to 3, as well as a physiologically acceptable vehicle.

**10.** Composition according to claim 9, **characterised in that** it is intended to be applied on the skin or to be administered orally.

**11.** Composition according to claim 10, **characterised in that** it is a cream, a gel, an oil, a lotion, a milk.

**12.** Composition according to claim 10, **characterised in that** it is a syrup, a pastille to be sucked, a capsule, a tablet.

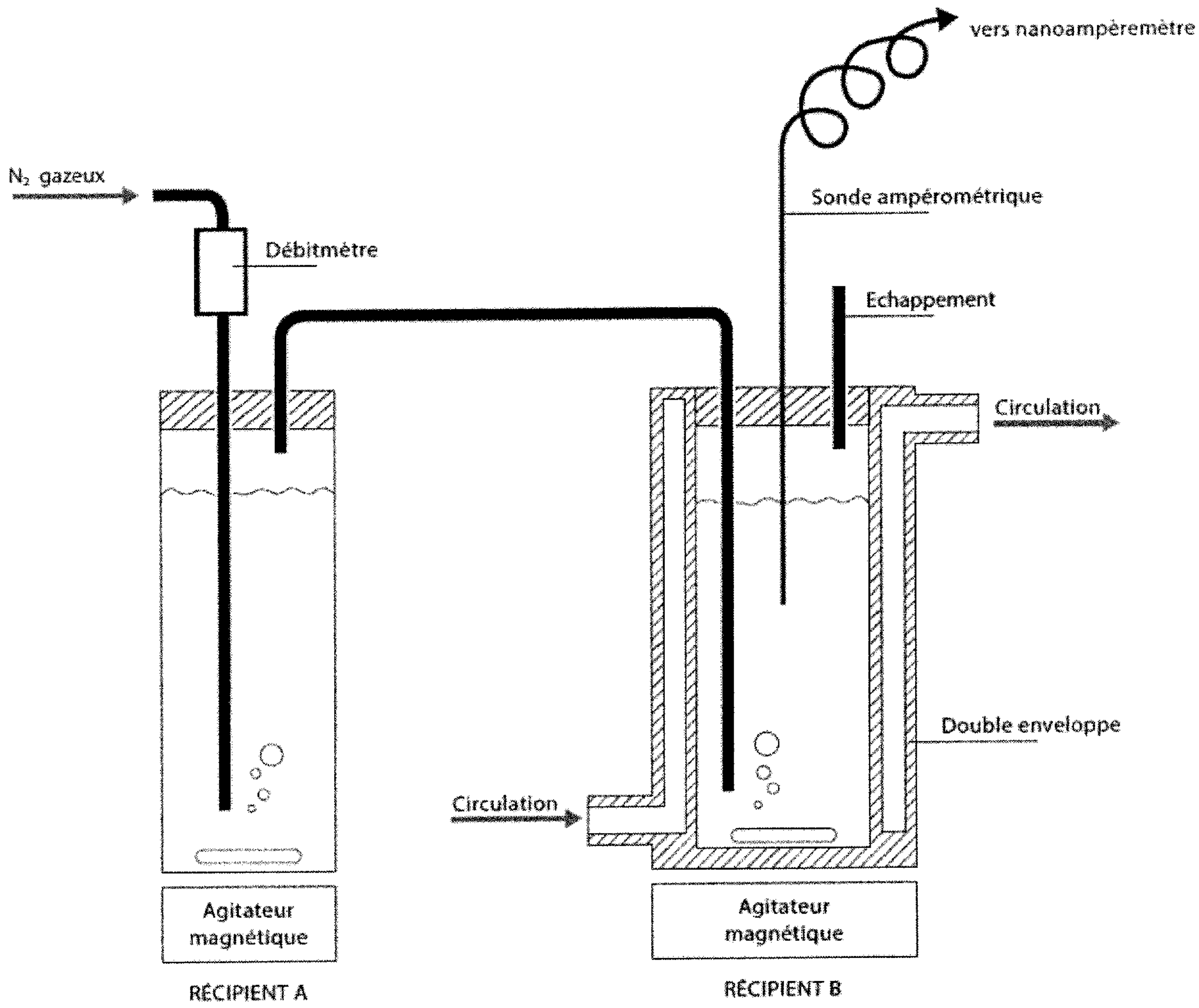
vers nanoampèremètre
$N_2$ gazeux
Sonde ampérométrique
Débitmètre
Echappement
Circulation
Double enveloppe
Circulation
Agitateur magnétique
Agitateur magnétique
RÉCIPIENT A
RÉCIPIENT B

Figure 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2002002292 W
- FR 2002002064 W
- WO 0207720 A
- FR 2733419


### Littérature non-brevet citée dans la description


- **R.M. PALMER ; D.S. ASHTON ; S. MONCADA.** Vascular endothelial cells synthetize nitric oxide from L-arginine. *Nature,* vol. 333 (6174), 664-666
- **R.M. PALMER ; A.G. FERRIGE ; S. MONCADA.** Nitric oxide release accounts for the biological activity of endothelium-derived relaxing factor. *Nature,* vol. 327 (6122), 524-526
- **M. A. MARIETTA ; P. S. YOON ; R. IYENGAR ; C. D. LEAF ; J. S. WISHNOK.** Macrophage Oxidation of L-Arginine to Nitrite and Nitrate: Nitric Oxide Is an Intermediate. *Biochemistry,* 1988, vol. 27, 8706-8711
- **J.B. HIBBS ; R.R. TAINTOR ; Z. VAVRIN ; E.M. RACHLIN.** Nitric oxide: a cytotoxic activated macrophage effector molecule. *Biochemical and Biophysical Communications,* 1988, vol. 157 (1), 87-94
- **S. MONCADA ; R.M.J. PALMER ; E.A. HIGGS.** Biosynthesis of nitric oxide from L-Arginine. A pathway for the regulation of cell function and communication. *Biochemical Pharmacology,* 1989, vol. 38 (11), 1709-1715
- **A. IALENTI ; A. IANARO ; S. MONCADA ; M. DI ROSA.** Modulation of acute inflammation by endogenous nitric oxide. *European Journal of Pharmacology,* 1992, vol. 211, 177-182
- **D. BRUCH-GERHARZ ; T. RUZICKA ; V. KOLB-BACHOFEN.** Nitric oxide and its implication in skin homeostasis and disease - A review. *Arch. Dermatol. Res.,* 1998, vol. 290, 643-651
- **N. S. TEKIN ; N. ILTER ; B. SANCAK ; M. G. OZDEN ; M. A. GURER.** Nitric Oxide Levels in Patients with Psoriasis Treated with Methotrexate. *Mediators of Inflammation,* 2006, vol. 2006, 1-5
- **A. H. A. MOHSEN ; H. A. WAHAB ; E. ALLAM.** New markers of disease activity in children with atopic dermatitis. *Journal of American Science,* 2011, vol. 7 (10), 404-408
- **M.-M. CALS-GRIERSON ; A.D. ORMEROD.** Nitric oxide function in the skin. *Nitric Oxide,* 2004, vol. 10, 179-193
- **K. A. KIRKEBOEN ; A. STRAND.** The role of nitric oxide in sepsis - an overview. *Acta Anaesthesiol. Scand.,* 1999, vol. 43, 275-288
- **H. MITSUHATA ; R. SHIMIZU ; M. M. YOKOYAMA.** Role of Nitric Oxide in Anaphylactic Shock. *Journal of Clinical Immunology,* 1995, vol. 15, 6
- **J. VÍTEČEK ; A. LOJEK ; G. VALACCHI ; L. KUBALA.** Arginine-Based Inhibitors of Nitric Oxide Synthase: Therapeutic Potential and Challenges. *Mediators of Inflammation,* 2012, vol. 2012, 1-22
- **W. STAHL ; M. MATEJOVIC ; P. RADERMACHER.** Inhibition of nitric oxide synthase during sepsis: revival because of isoform selectivity. *Shock,* 2010, vol. 34 (3), 321-322
- **CHIEKO MITAKA ; YUKIO HIRATA ; KUNINORI YOKOYAMA ; TAKASHI NAGURA ; YUKIO TSUNODA ; KEISUKE AMAHA.** Beneficial effect of carboxy-PTIO on hemodynamic and blood gas changes in septic shock dogs. *Crit Care,* 1997, vol. 1, 45
- **FRICKER SP ; SLADE E ; POWELL NA ; VAUGHAN OJ ; HENDERSON GR ; MURRER BA ; MEGSON IL ; BISLAND SK ; FLITNEY FW. BR.** Ruthenium complexes as nitric oxide scavengers: a potential therapeutic approach to nitric oxide-mediated diseases. *J. Pharmacol.,* 1997, vol. 122 (7), 1441-9
- **DAVIES R.** The synthesis and isolation of caffeoquinone and caffeoquinone methyl ester. *Tetrahedron letters,* 1976, vol. 4, 313-314
- **MATHIAS JOHN ; HANS GERD GUMBINGER ; HILKE WINTERHOFF.** Oxidation Products of Caffeic Acid as Model Substances for the Antigonadotropic Activity of Plant Extracts. *Planta Medica,* 1990, vol. 56, 14-18
- **DANIEL JANG ; GEORGE A. C. MURRELL.** Nitric oxide in arthritis. *Free Radical Biology & Medicine,* 1998, vol. 24 (9), 1511-1519
- **BOSKABADY M.H ; RAKHSHANDAH H. ; AFIAT M ; AELAMI Z ; AMIRI S. IRAN.** Antitussive effect of Plantigo lanceolate in Guinea Pigs. *J Med Sci.,* 2006, vol. 31 (3), 143-146